# EUROPÄISCHE PATENTSCHRIFT

(11) **EP 1 192 927 B1**
(45) Veröffentlichungstag und Bekanntmachung des Hinweises auf die Patenterteilung: **25.02.2009**
(21) Anmeldenummer: 01123022.4
(22) Anmeldetag: 26.09.2001
(51) Int. Cl.: A61J 1/20

(54) **Entnahmespike**
Withdrawal spike
Pointe pour prélèvement

(30) Priorität: 30.09.2000 DE 20016945 U
(43) Veröffentlichungstag der Anmeldung: 03.04.2002
(73) Patentinhaber: B. BRAUN MELSUNGEN AG, 34212 Melsungen (DE)
(72) Erfinder: Heil, Norbert, 34587 Felsberg (DE); Siemon, Bernd, 34212 Melsungen (DE); Siemon, Klaus, 34327 Körle (DE)
(74) Vertreter: Selting, Günther

(56) Entgegenhaltungen:
- WO-A-96/19154
- WO-A-99/08036
- DE-U- 29 913 550
- US-A- 4 128 098
- US-A- 5 269 771
- US-A- 5 527 306

## Beschreibung

Die Erfindung betrifft einen Entnahmespike zum Entnehmen medizinischer Flüssigkeiten aus Behältern.

Ein Entnahmespike, wie er beispielsweise in dem Patent DE 38 20 204 C2 beschrieben ist, weist einen Einstechdorn mit einem Belüftungskanal und einem Flüssigkeitskanal auf. Der Einstechdorn ist mit einem Griffteil verbunden. An diesem befindet sich ein mit dem Flüssigkeitskanal verbundener Anschlussstutzen. Bei der Benutzung des Entnahmespikes wird der Einstechdorn durch den Elastomerstopfen einer Flasche oder eines anderen Behälters hindurchgestochen. Dann wird an den Anschlussstutzen eine Spritze oder eine Schlauchleitung angeschlossen. Der Flüssigkeitsbehälter kann mit dem Kopf nach unten aufgehängt werden, wodurch bei der Entnahme Flüssigkeiten ausfließen und Luft in den Behälter eindringen kann. Der Entnahmespike ist auch zum Zuführen von Flüssigkeit zum Behälter geeignet.

Ein Entnahmespike wird von der Firma B. Braun Melsungen AG unter der Bezeichnung Mini-Spike Plus vertrieben. Bei diesem Entnahmespike ist der Anschlussstutzen von einem Schutzmantel umgeben, welcher einen aufklappbaren Deckel aufweist. Der verschließbare Schutzmantel dient einerseits als Kontaminationsschutz für den Anschlussstutzen und verhindert andererseits ein unkontrolliertes Austreten von Flüssigkeit in die Umgebung.

Bei der Entnahme von Heparin ist es üblich, alle Heparin-Spritzen der gesamten Krankenhausstation auf einmal aufzuziehen. Der Einstechdorn wird in eine Heparinflasche gestochen und diese wird zur Entnahme auf den Kopf gestellt. Die schon bereitliegenden Spritzen, die aus ihrer Sterilverpackung entnommen wurden, werden nacheinander an den Anschlussstutzen des Entnahmespikes angeschlossen und nach der Entnahme wieder in der jeweiligen Spritzenverpackung abgelegt. Während des Spritzenwechsels hält die ausführende Person die Flasche mit dem Kopf nach unten weiterhin in der Hand. Wenn der Spritzenwechsel nicht sehr schnell erfolgt, kann Heparin aus dem offenen Anschlussstutzen heraustropfen und die Umgebung verschmutzen.

Bei Zytostatikapräparaten, die toxisch sind, befürchtet das Personal stets, dass angebrochene Flaschen umfallen und dabei undicht werden könnten. Daher werden solche Flaschen häufig zusätzlich in einem PE-Beutel eingeschweißt. Der Schnappdeckel des Gerätes Mini-Spike Plus gilt allgemein als dicht und sicher.

In einigen Ländern ist es üblich Entnahmespikes in Verbindung mit Beuteln zu verwenden, indem man eine Perfusor-Spritze aus einem Beutel füllt. Dabei besteht die Gefahr des Nachtropfens.

Der Oberbegriff des Patentanspruchs 1 geht aus von einem Entnahmespike nach US-A-4128098. Dieser Entnahmespike enthält einen zylindrischen Schutzmantel mit einer Querwand. Die Querwand weist einen axialen Anschlussstutzen auf, durch den die stumpfe Spitze einer Spritze gesteckt werden kann. Die Spitze stößt dann gegen ein selbstschließendes Ventil, welches unterhalb der Querwand in einem Subgehäuse enthalten ist. Das selbstschließende Ventil ist ein ungelochter Elastomerkopf, der beim Öffnen von einem Ringwulst abhebt und damit einen Flüssigkeitskanal zu einem Einstechdorn öffnet. Dieser Flüssigkeitskanal führt außen an dem Elastomerkopf vorbei. Vor Benutzung ist der Anschlussstutzen durch einen eingesteckten Protektor geschlossen. Dieser steht in keiner Verbindung mit dem äußeren Schutzmantel.

In US-A-5269771 ist ein Entnahmespike beschrieben, der ein selbstschließendes Ventil mit einer an ihrem Rand eingespannten geschlitzten Ventilscheibe und einen gegen die Ventilscheibe drückenden rohrförmigen Ventilöffner aufweist. Durch Einsetzen der stumpfen Spitze einer Spritze wird der Ventilöffner vorgeschoben, um die Ventilscheibe aufzustoßen. Ein den Anschlussstutzen umgebender äußerer Schutzmantel ist ebenso wenig vorgesehen, wie ein verschließender Deckel.

Der Erfindung liegt die Aufgabe zugrunde, einen Entnahmespike zu schaffen, der ein Höchstmaß an Dichtigkeit und Anwendungssicherheit aufweist und ein hohes Maß an Sauberkeit, Hygiene und Komfort ermöglicht.

Die Lösung dieser Aufgabe erfolgt erfindungsgemäß mit den im Anspruch 1 angegebenen Merkmalen.

Der Anschlussstutzen enthält ein selbstschließendes Ventil, das durch Einführen eines Rohres in den Anschlussstutzen zu öffnen ist. Dadurch wird ein Nachtropfen mit Sicherheit verhindert. Bei dem erfindungsgemäßen Ventil ist die Funktion des Schutzmantels im Wesentlichen darauf beschränkt, eine Berührungskontamination des Anschlussstutzens zu verhindern.

Der Schutz gegen das Auslaufen von Flüssigkeit wird durch das Ventil erreicht, das nur dann im Öffnungszustand ist, wenn ein rohrförmiger Gegenstand in den Anschlussstutzen hineingedrückt wird.

Ventile, die durch Einführen eines rohrförmigen Gegenstandes öffnen, sind in der Medizintechnik bekannt, und zwar hauptsächlich bei Schlauchverbindungen wie Katheteranschlüssen.

Erfindungsgemäß ist ein selbstschließendes Ventil in den Anschlussstutzen eines Entnahmespikes integriert. Hierdurch wird ein nur unwesentlicher Mehraufwand hervorgerufen, der jedoch erhebliche Vorteile bei der Anwendung hervorruft. So wird beispielsweise der Spritzenwechsel beim Aufziehen von Heparinspritzen wesentlich erleichtert. Bei Zytostatikapräparaten wird die Gefahr, dass das Personal mit toxischen Flüssigkeiten in Berührung kommt, minimiert.

Berührung kommt, minimiert. Die Erfindung bietet auch besondere Vorteile beim Entnehmen von Flüssigkeiten aus Beuteln, weil hier die Gefahr des unkontrollierten Auslaufens besonders groß ist. Die Flüssigkeitssperre wirkt nicht erst am Auslass des Anschlussstutzens sondern bereits an dessen Einlass, so das auch die Innenwand des Anschlussstutzens von Kontaminationen freigehalten wird.

Im Folgenden wird unter Bezugnahme auf die Zeichnung ein Ausführungsbeispiel der Erfindung näher erläutert.

Es zeigen:
- Fig. 1: eine Seitenansicht des Entnahmespikes, teilweise geschnitten, und

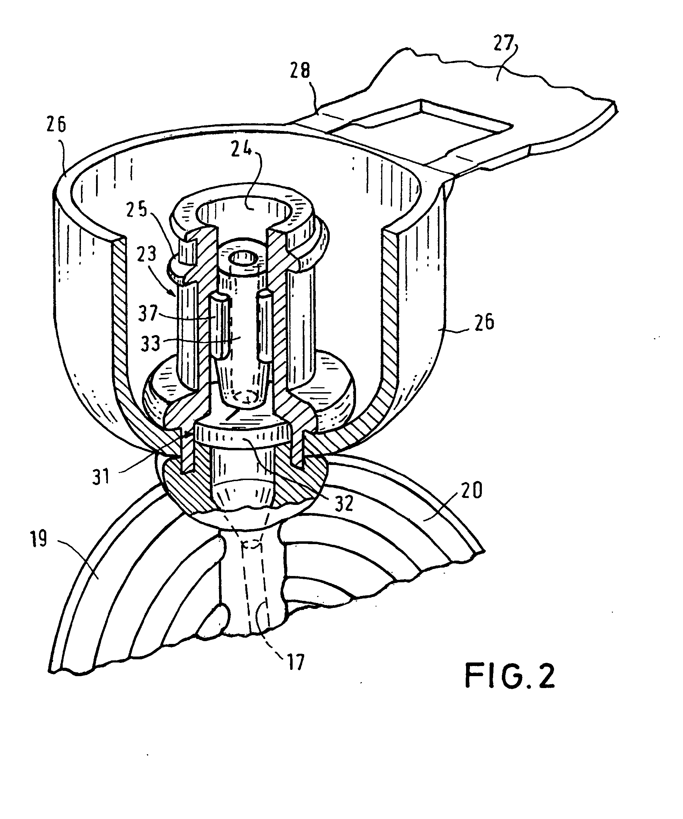

- Fig. 2: eine perspektivische Ansicht des Anschlussstutzens mit dem umgebenden Schutzmantel, teilweise geschnitten.

Der dargestellte Entnahmespike weist einen Einstechdorn 10 auf, der an einem Ende eine Spitze zum Durchstechen des Stopfens einer Flasche oder eines anderen Behältnisses hat und am gegenüberliegenden Ende einstückig mit einem Gehäuse 12 verbunden ist. Das Gehäuse 12 ist plattenförmig ausgebildet und es dient als Trägerplatte, von dem der Einstechdorn 10 mittig absteht. Das Gehäuse 12 enthält eine erste Filterkammer 13 mit einem Flüssigkeitsfilter und eine zweite Filterkammer 14 mit einem Luftfilter. Der Luftfilter hat eine Maschenweite von 0,45 µm und der Flüssigkeitsfilter ist ein Partikelfilter mit einer Maschenweite von 5 µm.

Auf dem Einstechdorn 10 sitzt eine abnehmbare Schutzkappe 40.

Durch den Einstechdorn 10 erstrecken sich in Längsrichtung ein Flüssigkeitskanal 15 und ein Luftkanal 16. Die beiden Kanäle 15,16 enden im Konusbereich der Spitze 11 des Einstechdornes. Im Innern des Gehäuses 12 sind die Kanäle 15 und 16 voneinander getrennt. Der Flüssigkeitskanal 15 steht mit der Filterkammer 13 in Verbindung und der Luftkanal 16 mit der Filterkammer 14. Die Filterkammer 13 ist ferner mit einem Kanal 17 verbunden, der durch ein Rohr 18 verläuft, welches in Verlängerung des Einstechdornes 10 mit dem Gehäuse 12 verbunden ist und zur entgegengesetzten Seite des Gehäuses absteht. An dem Rohr 18 greifen seitlich zwei Flügel 19,20 an, die jeweils als Viertelkreissektoren ausgebildet sind und sich zwischen dem Rohr 18 und dem Gehäuse 12 erstrecken. Die beiden Flügel 19,20 bilden insgesamt einen Halbkreis, der in einer Ebene liegt, die rechtwinklig zu der Plattenebene des Gehäuses 12 verläuft. An den Flügeln 19 und 20 sind auf beiden Seiten konzentrische Rippen 21 vorgesehen, die das Angreifen mit der Hand an den Rippen erleichtern. Die Flügel 19,20 bilden somit ein Griffteil und das plattenförmige Gehäuse 12 bildet eine Stoßfläche zum Angreifen mit der Hand beim Einstoßen des Einstechdornes 10 in einen Stopfen.

An dem Flügel 19 befindet sich eine Lüftungsöffnung 22, die mit der Filterkammer 14 in Verbindung steht. Im Strömungsweg der Luft befindet sich zwischen dem Luftkanal 16 und der Lüftungsöffnung 22 die in der Filterkammer 14 enthaltene Membran des Luftfilters.

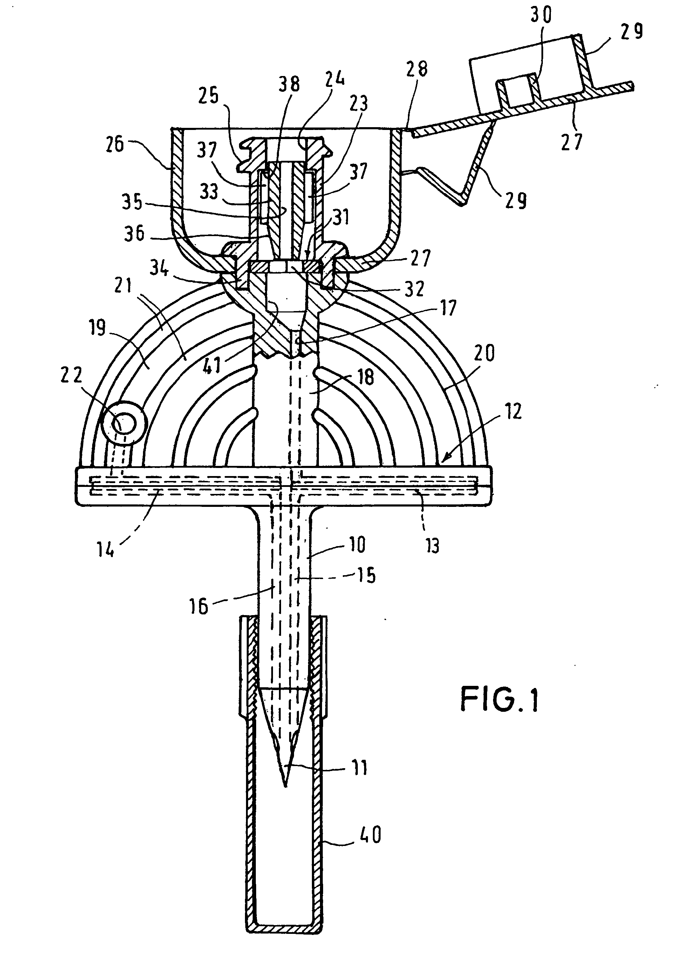

Auf dem Ende des Rohres 18 sitzt ein Anschlussstutzen 23 mit einem Innenkonus 24 und äußeren Gewinderippen 25 eines Luer-Lock-Verbinders. Der Anschlussstutzen 23 ist von einem Schutzmantel 26 ringförmig mit seitlichem Abstand umgeben. Der Schutzmantel 26 weist einen Boden 27 auf, der sich an den Fuß des Anschlussstutzens 23 abdichtend anschließt. Der Schutzmantel 26 überragt das äußere Ende des Anschlussstutzens 23. Am Rande des topfförmigen Schutzmantels ist ein klappbarer Deckel 27 mit einem Filmscharnier 28 befestigt. Der Deckel 27 ist ferner über einen Kniegelenkarm 29 mit dem Schutzmantel 26 verbunden. Der Kniegelenkarm 29 bewirkt ein Schnappverhalten des Deckels 27, der entweder die in Fig. 1 dargestellte Öffnungsstellung oder die Schließstellung einnimmt. An der Innenseite des Deckels ist ein vorspringender Rand 39 angeordnet, der der Schließstellung des Deckels passend in den Schutzmantel eingreift. Ferner ist an der Innenseite des Deckels ein zylindrisches Verschlussteil 30 vorgesehen, welches in den Innenkonus 24 des Anschlussstutzens 23 eindringt.

Im Innern des Anschlussstutzens 23 befindet sich das Ventil 31. Dieses weist eine Ventilscheibe 32 und einen Ventilöffner 33 auf. Die Ventilscheibe 32 aus Elastomermaterial ist zwischen dem Rand des Rohres 18 und einem Rand des Anschlussstutzens 23 an ihrem Rand eingespannt und wird von einer Muffe 34 des Anschlussstutzens übergriffen. Die Ventilscheibe 32 weist eine Schlitz- oder Öffnungsstruktur auf. Sie ist selbstschließend, d.h. ohne Einwirkung äußeren Drucks begibt sie sich in die in den Zeichnungen dargestellte Schließposition.

Der Ventilöffner 33 ist ein rohrförmiges Teil mit einem längslaufenden Kanal 35 und einem am Ende vorgesehenen Kegelstumpf 36, der gegen den Mittelteil der Ventilscheibe 32 stößt. An der Umfangsfläche des Ventilöffners 33 befinden sich nach außen abstehende Vorsprünge 37, die umfangsmäßig verteilt angeordnet sind. Die oberen Enden der Vorsprünge 37 stoßen gegen eine Ringschulter 38 im Innern des Anschlussstutzens 23. Über der Ringschulter 38 befindet sich der Innenkonus 24.

Unter der Ventilscheibe 32 befindet sich ein gegenüber dem Kanal 17 erweiterter Hohlraum 41, in den die Ventilscheibe bei Deformierung durch den Ventilöffner 33 ausweichen kann.

Bei Benutzung des Entnahmespikes wird ein männlicher Luer-Konus auf den Anschlussstutzen 23 aufgesetzt oder der Konus einer Spritze wird in den Innenkonus 24 eingeführt. Dabei stößt das eindringende Teil gegen die Stirnfläche des Ventilöffners 33, der dadurch in dem Anschlussstutzen 23 verschoben wird und die Ventilscheibe 32 aufdrückt. Dadurch wird das Ventil 31 zwangsweise im Öffnungszustand gehalten, solange das externe Teil in den Anschlussstutzen 23 hineinragt. Anschließend bewegt sich unter der Federwirkung der Ventilscheibe 32 der Ventilöffner 33 in die Ausgangsposition zurück und das Ventil 31 schließt von selbst wieder.

Flüssigkeitsreste, die sich noch im Anschlussstutzen 23 oder im Ventil 31 befinden könnten, werden durch das Verschließen des Deckels 27 am Austreten gehindert.

## Patentansprüche

1. Entnahmespike zum Entnehmen medizinischer Flüssigkeiten aus Behältern, mit
- einem Einstechdorn (10), der einen Belüftungskanal (16) und einen Flüssigkeitskanal (15) aufweist,
- einem mit dem Einstechdorn (10) verbundenen Griffteil (19,20),
- einem Anschlussstutzen (23), mit einem Einsteckkanal (24),
- einem den Anschlussstutzen (23) mit Abstand umgebenden Schutzmantel (26),
und einem im Flüssigkeitsweg enthaltenen selbstschließenden Ventil (31), das durch Einführen eines Rohres in den Anschlussstutzen (23) zu öffnen ist
**dadurch gekennzeichnet,**
**dass** das Ventil (31) eine an ihrem Rand eingespannte gelochte oder geschlitzte Ventilscheibe (32) und einen gegen die Ventilscheibe drückenden rohrförmigen Ventilöffner (33) aufweist, und
**dass** an dem Schutzmantel (26)ein klappbarer Deckel (27) angebracht ist, welcher den den Anschlussstutzen (23) enthaltenden Raum verschließt.

2. Entnahmespike nach Anspruch 1, **dadurch gekennzeichnet, dass** der Ventilöffner (33) an dem der Ventilscheibe (32) zugewandten Ende als Kegelstumpf (36) ausgebildet ist.

3. Entnahmespike nach Anspruch 1 oder 2, **dadurch gekennzeichnet, dass** der Ventilöffner (33) außen angeordnete Vorsprünge (37) aufweist, die gegen eine Ringschulter (38) des Anschlussstutzens (23) drücken.

4. Entnahmespike nach einem der Ansprüche 1-3, **dadurch gekennzeichnet, dass** der Deckel (27) ein Verschlussteil (30) enthält, welches in der Schließstellung des Deckels verschließend in den Anschlussstutzen (23) eingreift.

5. Entnahmespike nach einem der Ansprüche 1-4, **dadurch gekennzeichnet, dass** der Anschlussstutzen (23) als Luer-Lock-Verbinder ausgebildet ist.

## Claims

1. A withdrawal spike for withdrawing medical fluids from containers, comprising
- a piercing thorn (10) having a ventilation duct (16) and a fluid duct (15),
- a gripping part (19,20) connected with said piercing thorn (10),
- a connecting piece (23) provided with an insertion duct (24), and
- a protective jacket (26) surrounding said connecting piece (23) in a spaced relationship, and
- a self-closing valve (31) arranged in the fluid path, said valve being adapted to be opened by insertion of a tube into said connecting piece (23),
**characterized in that**
the valve (31) comprises a perforated or slotted valve disk (32) clamped-in at its edge, and a tubular valve opener (33) pressing against said valve disk, and
said protective jacket (26) comprising a hinged cover (27) which closes the space containing said connecting piece (23).

2. The withdrawal spike according to claim 1, wherein the valve opener (33) is configured as a truncated cone (36) at the side facing the valve disk (32).

3. The withdrawal spike according to claim 1 or 2, wherein the valve opener (33) comprises projections (37) arranged on the outside which press against an annular shoulder (38) of the connecting piece (23).

4. The withdrawal spike according to one of claims 1-3, wherein the cover (27) comprises a closing part (30) which, in the closed position, closingly engages with the connecting piece (23).

5. The withdrawal spike according to one of claims 1-4, wherein the connecting piece (23) is configured as a Luer-Lock connector.

## Revendications

1. Pointe pour prélèvement de liquides médicaux de récipients, comprenant
- un mandrin de perçage (10) avec un conduit d'aération (16) et un conduit pour liquide (15),
- un élément de poignée (19, 20) relié au mandrin de perçage (10),
- un raccord (23) avec un conduit d'insertion (24),
- une gaine protectrice (26) entourant ledit raccord (23) à une distance,
et une valve (31) à fermeture automatique prévue dans la voie du liquide, ladite valve étant apte à être ouverte par l'introduction d'un tuyau dans le raccord (23),
**caractérisée**
**en ce que** la valve (31) comprend un disque de valve (32) perforé et entaillé, serré par sa périphérie, et un moyen à ouverture de la valve (33) tubulaire pressant contre le disque de la valve, et
**en ce que** la gaine protectrice (26) est prévue d'un couvercle (27) pliable fermant l'espace contenant le raccord (23).

2. Pointe de prélèvement selon la revendication 1, **caractérisée en ce que** le moyen à ouverture de la valve (33) est en forme tronconique (36) à l'extrémité tournée vers le disque de valve (32).

3. Pointe de prélèvement selon la revendication 1 ou 2, **caractérisée en ce que** le moyen à ouverture de la valve (33) comprend des saillies (37) prévues à l'extérieur dudit moyen, pressant contre une épaule annulaire (38) du raccord (23).

4. Pointe de prélèvement selon une des revendications 1 - 3, **caractérisée en ce que** le couvercle (27) comprend un élément de fermeture (30) s'accrochant dans le raccord (23) d'une manière fermante quand le couvercle est dans la position fermeture.

5. Pointe de prélèvement selon une des revendications 1 - 4, **caractérisée en ce que** le raccord (23) est configuré comme raccord Luer-Lock.
